# EUROPÄISCHE PATENTSCHRIFT

(11) **EP 3 307 708 B1**
(45) Veröffentlichungstag und Bekanntmachung des Hinweises auf die Patenterteilung: **01.12.2021**
(21) Anmeldenummer: 16727505.6
(22) Anmeldetag: 07.06.2016
(51) Int. Cl.: C07C 263/10

(54) **VERFAHREN ZUR HERSTELLUNG VON DIISOCYANATEN IN DER GASPHASE**
METHOD FOR MANUFACTURING DIISOCYANATES IN THE GASEOUS PHASE
PROCÉDÉ DESTINÉ À LA FABRICATION DE DIISOCYANATES EN PHASE GAZEUSE

(30) Priorität: 12.06.2015 EP 15171968
(43) Veröffentlichungstag der Anmeldung: 18.04.2018
(73) Patentinhaber: Covestro Intellectual Property GmbH & Co. KG, 51373 Leverkusen (DE)
(72) Erfinder: LEHNER, Peter, 45481 Mülheim an der Ruhr (DE); BRUNS, Rainer, 51467 Bergisch Gladbach (DE); LORENZ, Wolfgang, 41540 Dormagen (DE)
(74) Vertreter: Levpat
(86) Internationale Anmeldenummer: PCT/EP2016/062905
(87) Internationale Veröffentlichungsnummer: WO 2016/198404

(56) Entgegenhaltungen:
- WO-A1-2010/010135
- US-A1- 2011 105 785

## Beschreibung

Die Erfindung betrifft ein Verfahren zur Herstellung von Diisocyanaten durch Umsetzung der entsprechenden primären Diamine mit Phosgen in der Gasphase, wobei der Druck im Diamin-Verdampfungsraum während der Überführung des Diamins in die Gasphase geringer ist als der Druck im Reaktionsraum während der Reaktion zu den Diisocyanaten und die Druckdifferenz zwischen Diamin-Verdampfungsraum und Reaktionsraum vor Vermischung von Diamin und Phosgen durch ein Druckerhöhungselement überwunden wird.

Diisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Diamine mit Phosgen. Eine Möglichkeit der Herstellung von Disocyanaten ist die Umsetzung der Diamine mit dem Phosgen in der Gasphase. Diese üblicherweise als Gasphasen-Phosgenierung bezeichnete Verfahrensführung zeichnet sich dadurch aus, dass die Reaktionsbedingungen so gewählt werden, dass zumindest die Reaktionskomponenten Diamin, Diisocyanat und Phosgen, bevorzugt jedoch sämtliche Edukte, Hilfsstoffe, Produkte und Reaktionszwischenprodukte, bei den gewählten Bedingungen gasförmig sind. Vorteile der Gasphasen-Phosgenierung sind u. a. ein verringerter Phosgen-Hold-Up, weniger Nebenprodukte, einfachere Aufarbeitung zum gewünschten Diisocyanat und erhöhte Reaktionsausbeuten. Die vorliegende Erfindung betrifft ausschließlich die Gasphasen-Phosgenierung.

Es sind aus dem Stand der Technik verschiedene Verfahren zur Herstellung von Isocyanaten durch Umsetzung von Aminen mit Phosgen in der Gasphase bekannt.

EP0 593 334B1offenbart ein Verfahren zur Herstellung von aromatischen Diisocyanaten in der Gasphase, wobei ein Rohrreaktor verwendet wird. Darin wird eine Vermischung der Edukte durch eine Einengung der Wände erreicht. Die Umsetzung erfolgt im Temperaturbereich von 250 °C bis 500 °C. Das Verfahren ist jedoch problematisch, da die Vermischung der Eduktströme allein über eine Verengung der Rohrwand im Vergleich zur Anwendung eines richtigen Mischorgans schlecht funktioniert. Eine schlechte Vermischung führt üblicherweise zu einer unerwünscht hohen Feststoffbildung. Bspw. können sich Harnstoffe aufgrund von einer Reaktion von bereits gebildetem Isocyanat mit noch nicht abreagiertem Ausgangsamin bilden.

Es hat nicht an Versuchen gefehlt, diese insbesondere bei der Umsetzung von aromatischen Diaminen mit Phosgen in der Gasphase zu beobachtende Bildung von Feststoffen zu minimieren, um so eine großtechnisch durchführbare Phosgenierung von aromatischen Diaminen in der Gasphase zu ermöglichen. Dabei fokussieren sich die Verbesserungen in dem Verfahren zur großtechnischen Phosgenierung von aromatischen Diaminen in der Gasphase auf die Verbesserung der Vermischung der Eduktströme sowie eine Vergleichmäßigung der Strömung in dem Gasphasenreaktor, die zu einer verlängerten Betriebszeit des Gasphasenreaktors führen. Neben schlechter Vermischung der Edukte trägt auch eine zu große thermische Belastung des aromatischen Diamins zu einer erhöhten Feststoffbildung bei, etwa durch Ammoniakabspaltung und nachfolgende Bildung von Ammoniumchlorid, das sich speziell bei der Rückgewinnung des im Überschuss eingesetzten Phosgen in den entsprechenden Apparaten niederschlagen kann.

EP0 570 799B1offenbart ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Verweilzeit von 0,5 bis 5 Sekunden durchgeführt wird, und bei dem die mittlere Abweichung von der mittleren Verweilzeit weniger als 6 % beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird. Wird die Rohrströmung durch eine Reynoldszahl oberhalb 4000 charakterisiert, ist nachteilig auch hier, dass wegen der notwendigen hohen Strömungsgeschwindigkeiten die Realisierung der zur vollständigen Umsetzung der Amine notwendigen Verweilzeit nur in sehr langen Misch- und Reaktorrohren möglich ist. Nach der Lehre von EP 0 570 799 B1 führen sowohl zu lange als auch zu kurze Verweilzeiten zu unerwünschter Feststoffbildung, so dass eine Vergleichmäßigung der Strömung in dem Reaktionsraum erforderlich und vor allem eine Rückvermischung der Komponenten in dem Reaktionsraum auszuschließen ist. EP 0 570 799 B1 offenbart, dass zur Erzeugung entsprechend kurzer Mischzeiten im Prinzip bekannte Methoden auf Basis von Mischaggregaten mit bewegten oder statischen Mischorganen, bevorzugt statischen Mischorganen, eingesetzt werden können, wobei nach der Lehre von EP 0 570 799 B1 insbesondere die Anwendung des Strahlmischerprinzips (Chemie-Ing.-Techn. 44 (1972) S. 1055, Abb. 10) ausreichend kurze Mischzeiten liefert. EP 0 570 799 B1 geht jedoch nicht näher auf die Bedingungen zur Verdampfung des Diamins und die Druckverhältnisse zwischen Verdampfungsraum und Reaktionsraum ein.

Maßnahmen zur Vergleichmäßigung der Strömungsverhältnisse sind ebenfalls Gegenstand von EP1 362 847B1**.** EP 1 362 847 B1 offenbart ein Verfahren zur Herstellung von aromatischen Diisocyanaten in der Gasphase, bei dem durch eine verbesserte Reaktionsführung in Rohrreaktoren durch strömungstechnische Maßnahmen wie der Vergleichmäßigung und Zentrierung der Eduktströme zeitliche Schwankungen der Temperatur und eine Asymmetrie in der Temperaturverteilung vermieden werden, die nach der Lehre von EP 1 362 847 B1 zu Anbackungen und Verstopfungen im Reaktor und damit zur Verkürzung der Betriebszeit der Reaktoren führen.

Nach der Lehre von EP1 449 826A1steht bei der Umsetzung der aromatischen Diamine mit Phosgen in der Gasphase die Umsetzung des Phosgens mit dem Diamin zu dem Diisocyanat in Konkurrenz zu der Folgereaktion des Diamins mit dem Diisocyanat zum entsprechenden Harnstoffoligomer, wobei eine verbesserte Mischung der Edukte Phosgen und Diamin bei gleichzeitiger Vermeidung von Rückströmung im Rohrreaktor die Selektivität der Diisocyanatbildung erhöht und die Harnstoffbildung reduziert. Dadurch lassen sich nach der Lehre von EP 1 449 826 A1 die Mengen an Kondensationsprodukt im Rohrreaktor verringern, die aufgrund ihrer Ablagerung an der Reaktorwand zu einer Verkleinerung des freien Rohrquerschnitts und zu allmählichem Druckanstieg im Reaktor führen und letztlich die Betriebszeit des Verfahrens bestimmen. Zur verbesserten Vermischung der Edukte Phosgen und Diamin offenbart EP 1 449 826 A1 den Einsatz einer sog. Mehrfachdüse. EP 1 449 826 A1 offenbart jedoch keine Details zur Überführung der Diamine in den gasförmigen Zustand und zu den Druckverhältnissen zwischen Verdampfungsraum und Reaktionsraum.

Apparative Lösungen zur verbesserten Mischung der Edukte offenbaren ebenfalls EP1 526 129A1**,** DE103 59 627A1 und WO2007/028 715A1**,** wobei strömungstechnische Maßnahmen zur Drallerzeugung (EP 1 526 129 A1 [e]), konzentrisch angeordnete Ringspaltdüsen mit singulärer (DE 103 59 627 A1[e],) oder mehrfacher Amineinspeisung (WO 2007/028 715 A1) wie auch mehrere, parallel zur Achse eines Rohrreaktors angeordnete Amindüsen (EP 1 449 826 A1) zum Einsatz kommen.

Gemäß der Lehre von EP-A-1 526 129 hat eine Erhöhung der Turbulenz des Eduktstromes in der Zentraldüse einen positiven Einfluss auf die Durchmischung der Reaktanden und damit auf die Gasphasenreaktion insgesamt. Als Folge der besseren Durchmischung nimmt die Neigung zur Nebenproduktbildung ab und die erforderliche Verweilzeit und somit Reaktorbaulänge sinken deutlich.

EP 1 526 129 A1 offenbart bei einem Einsatz einer Spiralwendel als Turbulenz erzeugendem Einbauelement in der Zentraldüse eine Verkürzung der Mischstrecke auf 42 % der ursprünglichen Länge. EP 1 526 129 A1 beschreibt zwar die Notwendigkeit, dass das Amin vor der Zuführung in den Reaktionsraum verdampft werden muss. Da allerdings keinerlei Daten zu den entsprechenden Druckverhältnissen offenbart werden, muss der Fachmann davon ausgehen, dass das Druckniveau im Verdampfungsraum höher ist als am Eintritt des Reaktionsraumes, um die gewünschte Stromführung zu realisieren.

Doch nicht nur die physikalisch-technischen Reaktionsbedingungen sondern ebenfalls die Eigenschaften der in die Umsetzung mit Phosgen in der Gasphase eingesetzten aromatischen Amine waren Gegenstand offenbarter Verfahren.

So müssen nach der Lehre von WO2008 / 071 564 A1 Amine, die in einer Gasphasenphosgenierung zu den entsprechenden Isocyanaten umgesetzt werden sollen, bestimmte Erfordernisse erfüllen. Danach sind speziell solche Amine geeignet, die sich während der Dauer der Reaktion unter den herrschenden Reaktionsbedingungen in dem Gasphasenreaktor zu höchstens 2 mol%, besonders bevorzugt zu höchstens 1 mol% und ganz besonders bevorzugt zu höchstens 0,5 mol% zersetzen. Nach der Lehre von WO 2008 / 071 564 A1 sind dies aliphatische oder cycloaliphatische Amine, es können aber auch aromatische Amine verwendet werden, sofern sich diese ohne signifikante Zersetzung in die Gasphase überführen lassen. Anweisungen, wie sich die als geeignet bezeichneten aromatischen Diamine ohne signifikante Zersetzung in die Gasphase überführen lassen oder wie eine Zersetzungsneigung wenigstens vermindert werden kann, sind der WO 2008 / 071 564 A1 jedoch nicht zu entnehmen.

Eine spezielle Verdampfungstechnik unter Beachtung der thermischen Belastung der in einer Gasphasenphosgenierung eingesetzten Amine offenbart EP 1 754 698 A2. Nach der Lehre von EP 1 754 698 A1 werden die in dem Reaktor zur Umsetzung der eingesetzten Amine mit Phosgen zu beobachtenden Ablagerungen zum Ersten durch die Zersetzung der eingesetzten Amine während der Reaktion verursacht. Zum Zweiten führen lange Verweilzeiten in der Verdampfung und Überhitzung speziell bei Einsatz von aliphatischen Aminen zu einer partiellen Zersetzung der eingesetzten Amine unter Ammoniakabspaltung. Diese speziell bei Einsatz von aliphatischen Aminen zu beobachtende partielle Zersetzung unter Ammoniakabspaltung während der Verdampfung vermindert nicht nur die Ausbeute, sondern es bilden sich bei der nachfolgenden Phosgenierungsreaktion auch Ablagerungen von Ammoniumchlorid in den nach geschalteten Rohrleitungen und Apparaten. Die Anlagen müssen dann relativ häufig gereinigt werden, wobei entsprechende Produktionsverluste entstehen. EP 1 754 698 A1 beschreibt, dass diese Nachteile insbesondere bei den üblicherweise für die Verdampfung und Überhitzung der Amine eingesetzten Rohrbündelwärmetauschern, Plattenwärmetauschern oder Fallfilmverdampfern auftreten. Als technische Lösung offenbart diese Schrift die Ammoniakabspaltung während der Verdampfung dadurch zu unterdrücken, dass für die Verdampfung und Überhitzung der aliphatischen Amine spezielle Milli- oder Mikrowärmetauscher eingesetzt werden sollten.

Nachteilig an der in der Schrift EP 1 754 698 A1 offenbarten Mikrowärmetauschern sind die sehr kleinen Kanäle, so dass bereits sehr kleine Feststoffmengen, die in technischen Prozessen stets vorhanden sind, zu einer Verstopfung führen, und somit die Betriebszeit der Verdampfers reduzieren. Weiterhin nachteilig an der offenbarten Totalverdampfung der Amine ist, dass die Amine keine nicht verdampfbaren Bestandteile enthalten dürfen, weil sich diese sonst zwangsläufig als fester Rückstand auf der Verdampferoberfläche ablagern, damit den Wärmeübergang verschlechtern und schließlich zur Verstopfung des Verdampfers führen. Die Bereitstellung von Aminen in der geforderten Qualität ist im technischen Prozess jedoch sehr aufwendig und teuer. Somit wird durch die Lehre dieser Schrift zwar die Betriebszeit des Reaktors verbessert, die Betriebszeit des Verdampfers jedoch deutlich verschlechtert, so dass die gesamte Betriebszeit der Produktionsanlage nicht vorteilhaft verbessert wird.

Die Minimierung der thermischen Belastung der Amine bei ihrer Verdampfung zur Umsetzung mit Phosgen in der Gasphase ist ebenfalls Gegenstand von EP1 935 876A1**.** EP 1 935 876 A1 offenbart, dass die Amine vor der Umsetzung mit Phosgen in der Regel verdampft und auf 200 °C bis 600 °C erhitzt und gegebenenfalls verdünnt mit einem Inertgas wie N₂, He, Ar oder mit den Dämpfen eines inerten Lösungsmittels, z. B. aromatischen Kohlenwasserstoffen ggf. mit Halogensubstitution, wie z. B. Chlorbenzol oder o-Dichlorbenzol, dem Reaktionsraum zugeführt werden. Nach Lehre dieser Schrift kann die Verdampfung der Ausgangsamine in allen bekannten Verdampfungsapparaturen erfolgen, bevorzugt werden Verdampfungssysteme eingesetzt, bei denen ein kleiner Arbeitsinhalt mit einer hohen Umwälzleistung über einen Fallfilmverdampfer geführt wird und dabei zur Minimierung der thermischen Belastung der Ausgangsamine der Verdampfungsvorgang ggf. unter Zuspeisung von Inertgas und / oder Dämpfen eines inerten Lösungsmittels unterstützt wird.

WO2010/010 135A1offenbart ein Verfahren, bei dem die Edukte Amin und Phosgen mittels eines Ejektors in eine Mischzone überführt werden, an welche sich ein Diffusor zur Erhöhung von Druck und Temperatur anschließt. Dies erlaubt es, das Amin bei einem geringeren Druck als im Reaktor herrscht zu verdampfen, wodurch die Siedetemperatur des Amins herabgesetzt wird. Nachteilig bei diesem Verfahren ist, dass die Druckabsenkung im Aminverdampfer stark vom Mengenverhältnis Amin : Phosgen und somit vom Phosgenüberschuss abhängig ist. Somit hat eine Änderung der Reaktionsbedingungen immer auch Einfluss auf den Druck im Aminverdampfer und die dort herrschende Temperatur.

Es wäre wünschenswert, den Amin- und Phosgenstrom in weiten Grenzen unabhängig voneinander einstellen zu können, um Reaktionsführung und Aminverdampfung unabhängig voneinander beeinflussen zu können, wie dies zum Beispiel bei Änderung des dem Reaktor zugeführten Diamin-Mengenstroms notwendig ist. Insbesondere wäre eine Verfahrensführung wünschenswert, die es ermöglicht
- die jeweiligen Inertgasanteile in den Eduktströmen frei und unabhängig voneinander einstellen zu können, um zum Beispiel unnötigen Gasballast und eine aufwändige Ausschleusung bzw. Rückführung zu vermeiden und um auch bei sich ändernden Diamin-Mengenströmen zum Reaktionsraum hin immer im günstigsten Bereich der Fluidströmung arbeiten zu können,
- den Phosgenvordruck bei gegebenem Druck im Reaktionsraum so gering wie möglich halten zu können, um den Sicherheitsanforderungen im Umgang mit Phosgen besser gerecht zu werden, und
- den regeltechnischen Aufwand der Eduktsteuerung aus Kosten- und Verfügbarkeitsgründen so einfach wie möglich zu halten.

Trotz der vielen Versuche, die Umsetzung von Aminen mit Phosgen in der Gasphase zu optimieren und die dabei häufig zu beobachtende Bildung von Feststoffen zu minimieren, besteht daher ein weiterer Bedarf zur Verbesserung der Gasphasenphosgenierung von Aminen.

Diesem Bedarf Rechnung tragend ist ein Gegenstand der vorliegenden Erfindung die Bereitstellung eines Verfahrens zur Gasphasenphosgenierung von primären Diaminen, das die Überführung des zu phosgenierenden Diamins in die Gasphase möglichst schonend ausgestaltet und in der Wahl der Prozessparameter möglichst viele Freiheitsgrade ermöglicht.

Gegenstand der vorliegenden Erfindung ist dabei insbesondere ein Verfahren zur Herstellung eines Diisocyanats durch Umsetzung des korrespondierenden primären Diamins mit Phosgen in der Gasphase, worin
(i) das primäre Diamin getrennt vom Phosgen bei einem Druck p1 in die Gasphase überführt wird, sodass ein gasförmiger, das Diamin enthaltender Eduktstrom A erhalten wird,
(ii) der in (i) erhaltene Eduktstrom A durch ein Druckerhöhungselement geführt wird, in dem der Druck des Eduktstroms A auf einen Wert p2>pl erhöht wird, wobei die Druckdifferenz p2 ― p1 bevorzugt 50 mbar bis 1500 mbar beträgt.
(iii) ein Phosgen enthaltender gasförmiger Eduktstrom P, der unter dem Druck p3 steht, in einer Mischvorrichtung mit dem Strom A aus Schritt (ii) vermischt wird, wobei p3 bevorzugt größer als oder gleich p2, besonders bevorzugt größer als und ganz besonders bevorzugt um 10 mbar bis zu 100 mbar größer als p2 ist, und
(iv) der in (iii) erhaltene Mischstrom aus den Eduktströmen A und P in einem Reaktionsraum bei einem Druck p4 zum Diisocyanat umgesetzt wird, wobei p4 bevorzugt kleiner als p2 und kleiner als p3 ist.

Unter dem Begriff *primäres Diamin* werden erfindungsgemäß sowohl aliphatische als auch aromatische primäre Diamine zusammengefasst.

Sämtliche *Druckangaben* beziehen sich auf absolute Drücke.

Unter *Umsetzung in der Gasphase* ist zu verstehen, dass die Diamine im gasförmigen Zustand zu den Diisocyanaten reagieren und im Verlauf der Reaktion sämtliche vorhandenen Komponenten (Edukte, Produkte, Zwischenprodukte, ggf. Nebenprodukte, ggf. Inertstoffe) während des Durchgangs durch den Reaktionsraum zu mindestens 95,0 Massen-%, bevorzugt zu mindestens 98,0 Massen-%, besonders bevorzugt zu mindestens 99,0 Massen-%, ganz besonders bevorzugt zu mindestens 99,8 Massen-% und speziell zu mindestens 99,9% jeweils bezogen auf die Gesamtmasse aller vorhandenen Komponenten, in der Gasphase verbleiben.

Unter *Reaktionsraum* wird dabei der Raum verstanden, in welchem die Voraussetzungen für eine Gasphasenreaktion von primärem Diamin (bzw. Zwischenprodukten) mit Phosgen zum gewünschten Diisocyanat gegeben sind. Der Reaktionsraum beginnt also an der Stelle, an welcher der gasförmige Diaminstrom und der gasförmige Phosgenstrom zum ersten Mal miteinander unter Bedingungen, die eine Reaktion ermöglichen, in Kontakt treten und endet an der Stelle, an der entweder der aus Produkten, Nebenprodukten, ggf. nicht umgesetzten Edukten und Zwischenprodukten sowie ggf. zugesetzten inerten Stoffen bestehende Gasstrom in eine Vorrichtung zur Verflüssigung des gebildeten Diisocyanats (Kondensations- oder Quenchstufe) geführt oder die Reaktion durch Einleiten anderer geeigneter Maßnahmen (z. B. plötzliche Temperaturerniedrigung) abgebrochen wird. Der Reaktionsraum befindet sich in einer technischen Vorrichtung zur Durchführung von chemischen Reaktionen, dem *Reaktor.* Im einfachsten Fall ist der Reaktionsraum identisch mit dem Innenvolumen des Reaktors.

Die *Überführung des primären Diamins in die Gasphase* erfolgt in einem *Verdampfungsraum.* Der Verdampfungsraum befindet sich in einer technischen Vorrichtung zur Verdampfung von Flüssigkeiten, dem *Verdampfer.* Im einfachsten Fall ist der Verdampfungsraum identisch mit dem Innenvolumen des Verdampfers.

Unter einem *Druckerhöhungselement,* wird im Rahmen dieser Erfindung eine technische Vorrichtung zur Erhöhung des Drucks p1 auf den Wert p2 verstanden. Drücke werden im Rahmen dieser Erfindung bevorzugt mittels handelsüblicher, dem Fachmann bekannten Drucktransmitter gemessen.

Nachfolgend wird die Erfindung im Detail erläutert. Verschiedene Ausführungsformen der Erfindung sind dabei beliebig miteinander kombinierbar, sofern sich für den Fachmann aus dem Kontext nicht eindeutig das Gegenteil ergibt.

Bei dem erfindungsgemäßen Verfahren werden bevorzugt solche primären Diamine verwendet, die problemlos ohne Zersetzung in die Gasphase überführt werden können oder bei denen eine inhärent vorhandene Zersetzungsneigung durch Anwendung des erfindungsgemäßen Verfahrens auf ein akzeptables Maß zurückgedrängt werden kann. Ein "akzeptables Maß" bedeutet in diesem Zusammenhang, dass bevorzugt weniger als 1 mol%, besonders bevorzugt weniger als 0,7 mol% und ganz besonders bevorzugt weniger als 0,1 mol% des primären Diamins bei der Überführung in die Gasphase zersetzt werden.

Sowohl aliphatische bzw. cycloliphatische (d. h. Diamine, bei denen die Aminogruppe(n) an Kohlenstoffatome einer aliphatischen oder cycloaliphatischen Struktur gebunden ist/sind) als auch aromatische Amine (d. h. Amine, bei denen die Aminogruppe(n) an Kohlenstoffatome einer aromatischen Struktur gebunden ist/sind) sind für das erfindungsgemäße Verfahren geeignet.

Aus der Reihe der aliphatischen oder cycloaliphatischen Diamine sind besonders solche geeignet, die 1 bis 15 Kohlenstoffatome besitzen. Bevorzugt werden 1,6-Diamino-hexan, 1-Amino-3,3,5-trimethyl-5-aminomethylcyclohexan (IPDA) und 4,4'-Diaminodicyclohexylamin verwendet. Besonders bevorzugt verwendet wird 1,6-Diaminohexan (HDA).

Aus der Reihe der aromatischen Diamine sind besonders solche geeignet, die ein bis zwei aromatische Ringe enthalten. Beispiele für bevorzugte aromatische Diamine sind Toluylendiamin (TDA), insbesondere 2,4-TDA und 2,6-TDA und Gemische daraus, Diaminobenzol (alle Isomere), Naphthyldiamin (NDA, alle Isomere) und 2,2'-, 2,4'- oder 4,4'-Methylendiphenyldiamin (MDA) oder Isomerengemische davon. Besonders bevorzugt ist Toluylendiamin (TDA), insbesondere sind 2,4-TDA und 2,6-TDA und deren Gemische bevorzugt.

Die Ausgangsdiamine werden im erfindungsgemäßen Verfahren zunächst in einem Verdampfungsraum in die Gasphase überführt **(Schritt (i)**)**.** Dies geschieht bevorzugt mit Hilfe von mindestens einer Verdampfungsvorrichtung wie aus dem Stand der Technik, zum Beispiel aus EP-A-2196455, bekannt. Die Diamine werden auf 180 °C bis 600 °C, bevorzugt 190 °C bis 500 °C, besonders bevorzugt 200 °C bis 450 °C erhitzt, gegebenenfalls mit einem Inertgas wie N₂, He, Ar oder mit den Dämpfen eines inerten Lösungsmittels, z. B. aromatischen Kohlenwasserstoffen, ggf. mit Halogensubstitution, wie z. B. Chlorbenzol oder o-Dichlorbenzol, verdünnt und dem Reaktionsraum zugeführt. Dabei besteht der Eduktstrom A bevorzugt zu 99,95 mol-% bis 75 mol-% aus dem zu phosgenierenden Diamin und zu 0,05 mol-% bis 25 mol-% aus einem Inertgas, welches besonders bevorzugt Stickstoff ist.

Erfindungsgemäß liegt das Druckniveau im Verdampfungsraum für das Diamin bei einem Wert p1, der geringer ist als der Druck p2, unter dem das Diamin bei Eintritt in die Mischvorrichtung steht. Der Druck p2 muss mindestens so groß sein wie der im Reaktionsraum herrschende Druck (nachfolgend p4). Der Druck p4 wird bevorzugt am Eintritt in den Reaktionsraum gemessen. Da bei Durchlaufen der Mischvorrichtung oft ein apparativ bedingter Differenzdruck zu überwinden ist (Druckverlust über die Mischeinrichtung), ist es in einem solchen Fall erforderlich, p2 (entsprechend der Größe des Druckverlusts über die Mischeinrichtung) größer als p4 zu wählen. Bevorzugt liegt der Druck p1 50 mbar bis 1500 mbar, besonders bevorzugt 75 mbar bis 1400 mbar und ganz besonders bevorzugt 100 mbar bis 1250 mbar unterhalb des Druckes p2. Unter dem Druck p1,bei dem das Diamin in die Gasphase überführt wird, wird der von Verdampfungsraum bis Eingang Druckerhöhungselement herrschende Druck verstanden. Dieser Druck p1 wird bevorzugt am Eingang des Druckerhöhungselements gemessen.

Bevorzugt beträgt der Druck bei der Überführung des Diamins in die Gasphase (p1) 50 mbar bis 1500 mbar. Im Rahmen der vorliegenden Erfindung kann die Überführung des Diamins in die Gasphase grundsätzlich zwar sowohl bei gegenüber Normaldruck (1013 mbar) vermindertem als auch erhöhtem Druck durchgeführt werden. Je kleiner jedoch p1 ist, desto geringer ist die Wahrscheinlichkeit von Zersetzungsreaktionen, sodass möglichst kleine Drücke p1 zu bevorzugen sind. Andererseits kann p1 nicht beliebig klein gewählt werden, weil sonst die zu überwindende Druckdifferenz p2 - p1 zu groß würde. Daher sind die in der industriellen Praxis zu wählenden Drücke p1 bestimmt durch die konkreten Randbedingungen (Art des Diamins, Art des Druckerhöhungselements, ggf. vorhandener Druckverlust über die Mischeinrichtung, optimaler Reaktionsdruck p4). Im Fall von TDA als dem zu phosgenierendem Diamin hat sich ein bevorzugter Druck p1 von 50 mbar bis 1500 mbar, besonders bevorzugt von 75 mbar bis 1400 mbar, ganz besonders bevorzugt von 100 mbar bis 1250 mbar bewährt, wobei der optimale Reaktionsdruck p4 bevorzugt 80 bis 2500 mbar, besonders bevorzugt 120 mbar bis 2400 mbar und ganz besonders bevorzugt 150 mbar bis 2200 mbar beträgt.

Die Überführung der Diamine in die Gasphase in einem Verdampfungsraum kann in allen aus dem Stand der Technik bekannten Verdampfertypen erfolgen. Bevorzugt werden solche Verdampfertypen eingesetzt, bei denen ein kleiner Arbeitsinhalt mit einer hohen Umwälzleistung über einen Fallfilmverdampfer geführt wird und dabei zur Minimierung der thermischen Belastung der Ausgangsdiamine der Verdampfungsvorgang - wie oben ausgeführt - ggf. durch Zuspeisung von Inertgas und / oder Dämpfen eines inerten Lösungsmittels unterstützt wird. Bevorzugt können auch Rohrbündelwärmetauscher oder Plattenwärmetauscher, gegebenenfalls mit Umpumpkreislauf, eingesetzt werden.

In einer weiteren Ausführungsform des erfindungsgemäßen Verfahrens können auch Verdampfungsapparaturen eingesetzt werden, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Diaminen wird z. B. in EP 1 754 698 A2 offenbart. Bevorzugt werden im Rahmen dieser Erfindung die in den Absätzen [0007] bis [0008] und [0017] bis [0039] der in EP 1 754 698 A2 offenbarten Apparate eingesetzt.

Die gasförmigen Diamine können noch Anteile an unverdampften Tröpfchen der Diamine enthalten, d. h. als Aerosole vorliegen. Bevorzugt enthalten die gasförmigen Diamine jedoch im Wesentlichen keine Tröpfchen an unverdampften Diaminen. In diesem Zusammenhang bedeutet "im Wesentlichen", dass maximal 0,5 Massen-% des Diamins, besonders bevorzugt maximal 0,05 Massen-% des Diamins, bezogen auf die Gesamtmasse des Diamins, in Form von unverdampften Tröpfchen vorliegen und der restliche Teil des Diamins gasförmig vorliegt. Ganz besonders bevorzugt enthalten die gasförmigen Diamine keine Tröpfchen an unverdampften Diaminen.

In einer bevorzugten Ausführungsform wird das gasförmige Diamin nach dem Austritt aus dem Verdampfer und vor Durchlaufen des Druckerhöhungselements überhitzt. Dabei wird die Temperatur, die das gasförmige Diamin bei Austritt aus dem Verdampfer hat (T1) auf einen Wert T11 erhöht, und zwar bevorzugt um bis zu 150 °C, besonders bevorzugt um bis zu 120 °C, ganz besonders bevorzugt um bis zu 90 °C. Dadurch wird sichergestellt, dass die Temperatur des gasförmigen Diamins signifikant oberhalb seines Taupunkts liegt. Dadurch wird vermieden, dass Teile des gasförmigen Diamins auf dem Weg vom Verdampfer zum Druckerhöhungselement, im Druckerhöhungselement oder auf dem Weg vom Druckerhöhungselement zum Reaktionsraum kondensieren. Durch Kondensation würden sich Flüssigkeitstropfen bilden, die zu Anbackungen an den Wänden oder Beschädigungen im Druckerhöhungselement führen können. Die Überhitzung bereits gasförmig vorliegenden Diamins kann bei entsprechend geringer Verweilzeit ohne große Gefahr von Zersetzungsreaktionen durchgeführt werden. Im Druckerhöhungselement selbst wird dann der Druck des Diamins auf p2 gesteigert, wobei es zu einer weiteren, im Vergleich zur Erhöhung von T1 auf T11 in der Regel geringeren, Temperaturerhöhung auf den Wert T2 kommt.

Weiterhin erfolgt die Verdampfung und Überhitzung des Ausgangsdiamins bevorzugt mehrstufig, um unverdampfte Tröpfchen im gasförmigen Eduktstrom A zu vermeiden. Bevorzugt sind Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z. B. beschrieben in "Droplet Separation", A. Bürkholz, VCH Verlagsgesellschaft, Weinheim - New York - Basel - Cambridge, 1989. Nach Verlassen des in Strömungsrichtung letzten Überhitzers wird der auf seine Solltemperatur vorgeheizte gasförmige Eduktstrom A dem Druckerhöhungselement zugeführt. Nach Verlassen des in Strömungsrichtung letzten Überhitzers wird der auf seine Solltemperatur vorgeheizte gasförmige Eduktstrom A mit einer mittleren Verweilzeit zwischen Austritt aus dem letzten Überhitzer und Eintritt in den Reaktionsraum von bevorzugt 0,01 s bis 60 s, ganz besonders bevorzugt von 0,01 s bis 30 s, insbesondere bevorzugt 0,01 s bis 15 s, dem Reaktionsraum zur Umsetzung zugeführt.

Da der Druck im Reaktionsraum p4 erfindungsgemäß größer ist als der Druck im Verdampfungsraum p1, muss der Eduktstrom A zur Förderung in den Reaktionsraum (und zuvor durch die Mischvorrichtung) ein Druckerhöhungselement durchlaufen **(Schritt (ii)**). Dieses ist bevorzugt ein Verdichter. Denkbar sind hier alle dem Fachmann bekannten Vorrichtungen zur Komprimierung und Förderung von Prozessgasen, wie z. B. Kolbenverdichter, Schraubenverdichter, Drehkolbenverdichter oder Turboverdichter. Geeignete Verdichter sind beispielsweise beschrieben in: "Pre-Designed Turboverdichter (Turbogebläse)", Herausgeber und Copyright 2010: Siemens AG, Energy Sector Freyeslebenstraße 1, 91058 Erlangen, Deutschland. Bevorzugt ist ein Verfahren, bei dem das Druckerhöhungselement, das der Eduktstrom A durchläuft, ausgewählt ist aus der Gruppe bestehend aus:
Kolbenverdichter, Schraubenverdichter, Drehkolbenverdichter und Turboverdichter.

Bevorzugt wird der Eduktstrom A im Druckerhöhungselement auf einen Druck p2 von 100 mbar bis 3000 mbar, besonders bevorzugt von 150 mbar bis 2800 mbar, ganz besonders bevorzugt von 200 mbar bis 2500 mbar verdichtet Bevorzugt beträgt die Druckdifferenz p2 - p4 20 mbar bis 800 mbar, besonders bevorzugt 20 mbar bis 500 mbar, ganz besonders bevorzugt 30 mbar bis 400 mbar und außerordentlich ganz besonders bevorzugt 50 mbar bis 300 mbar.

Die erfindungsgemäße Vorgehensweise aus (a) Überführung des Diamins in die Gasphase bei einem relativ niedrigem Druck p1 (und damit relativ niedriger Temperatur), (b) ggf. Überhitzung des erhaltenen Eduktstroms A und (c) Verdichtung des Eduktstroms A auf einen Druck p2 ≥ p4 ≥ p1 ist für das zu phosgenierende Diamin wesentlich schonender als die sofortige Überführung des Diamins in die Gasphase bei einem Druck p2.

Zur Bereitstellung des phosgenhaltigen Eduktstroms P in **Schritt (iii)** wird Phosgen bei einem Druck p3 auf Temperaturen von 200 °C bis 600 °C erhitzt und gegebenenfalls verdünnt mit einem Inertgas wie N₂, He, Ar oder mit den Dämpfen eines inerten Lösungsmittels, z. B. aromatischen Kohlenwasserstoffen ohne oder mit Halogensubstitution, wie z. B. Chlorbenzol oder o-Dichlorbenzol. Die zuvor genannten Verdünnungsmittel können auch schon während der Erhitzung des Phosgens zugegen sein. Der so erhaltenene Eduktstrom P, der unter dem Druck p3 steht, wird der Mischvorrichtung zugeführt. Bevorzugt umfasst der Eduktstrom P von 99,95 mol-% bis 75 mol-% Phosgen und von 0,05 mol-% bis 25 mol-% Inertgas oder Dämpfe eines inerten Lösungsmittels. Besonders bevorzugt wird ein Inertgas eingesetzt, ganz besonders bevorzugt Stickstoff. Der Eduktstrom P wird der Mischvorrichtung bevorzugt ohne vorhergehende Verdichtung zugeführt. Der Druck p3, unter dem der Eduktstrom P steht, beträgt bevorzugt von 100 mbar bis 3000 mbar, besonders bevorzugt von 150 mbar bis 2800 mbar und ganz besonders bevorzugt von 200 mbar bis 2500 mbar. Die Druckdifferenz p3 - p4 beträgt bevorzugt 20 mbar bis 800 mbar, besonders bevorzugt 20 mbar bis 500 mbar, ganz besonders bevorzugt 30 mbar bis 400 mbar und außerordentlich ganz besonders bevorzugt 50 mbar bis 300 mbar.

Die Mischung der Eduktströme A und P in der Mischvorrichtung kann auf verschiedene Weisen realisiert werden. Unabhängig von der Ausgestaltung der Mischvorrichtung im Detail wird bei der Vermischung und der weiteren Reaktion im Reaktionsraum über technische Maßnahmen, z. B. eine ausreichende Isolierung zur Vermeidung von Abstrahlungsverlusten, der Gefahr einer erneuten Tröpfchenbildung begegnet. Die Mischvorrichtung wird häufig einen apparativen Differenzdruck Δp_{Mischung} aufweisen, d. h. p2 und p3 müssen in einem solchen Fall jeweils mindestens gleich der Summe aus p4 (Druck im Reaktionsraum) und dem apparativen Differenzdruck Δp_{Mischung} sein.

Zur Durchführung kurzer Mischzeiten sind beispielsweise Mischaggregate oder Mischzonen mit bewegten oder statischen Mischorganen (bspw. Düsen) geeignet. Bevorzugt sind statische Mischer in Mischzonen, wie sie zum Beispiel in EP-B-1 362 847, EP-B-1 526 129 oder EP-B-1 555 258 beschrieben und der Gruppe der Glattstrahldüsen und Düsen mit turbulenzerzeugenden Elementen zuzuordnen sind. Bevorzugt werden in dem erfindungsgemäßen Verfahren die in den Absätzen [0008] bis [0014] und [0023] bis [0026] der EP 1362847, die in den Absätzen [0008] bis [0013] und [0022] bis [0027] der EP 1526129 oder die in den Absätzen [0007] bis [0011] und [0020] bis [0027] der EP-B-1 555 258 offenbarten Apparate eingesetzt. Alternativ zu den genannten Mischaggregaten können auch die in EP-A-1 449 826 in den Abschnitten [0011], [0012] und [0019] bis [0027] offenbarten mehreren, parallel zur Achse des Rohrreaktors angeordneten Düsen (Mehrfachdüsen) eingesetzt werden.

Besonders bevorzugt kommen Reaktoren mit im Wesentlichen rotationssymmetrischen Reaktionsräumen zum Einsatz, bei denen die gasförmigen Edukte, ggf. verdünnt mit Inerten, dem zumindest einen Mischraum nach dem Strahlmischerprinzip (Chemie-Ing. Techn. 44 (1972) S. 1055, Abb. 10) zugeführt werden. Bevorzugt treten dabei die eingespeisten Stoffströme mit einem Geschwindigkeitsverhältnis von 2-20, besonders bevorzugt 3-15, ganz besonders bevorzugt 4-12, in den zumindest einen Mischraum der Reaktoren ein. Bevorzugt wird dabei dem zumindest einen Mischraum der Reaktoren das Amin, ggf. verdünnt mit Inerten, mit der höheren Strömungsgeschwindigkeit zugeführt.

In **Schritt (iv)** erfolgt die weitere Umsetzung der in Schritt (iii) erhaltenen Mischung aus Amin und Phosgen zum Diisocyanat im Reaktionsraum. Bevorzugt weisen weder der Reaktionsraum (noch die Mischvorrichtung) Heizflächen auf, die Anlass zu einer thermischen Belastung mit der Folge von Folgereaktionen wie Isocyanurat- oder Carbodiimidbildung Anlass geben können, noch Kühlflächen auf, die bevorzugt Anlass zu einer Kondensation mit der Folge von Ablagerung hochsiedender Polymerkomponenten geben können. Die Edukte Phosgen und Diamin werden so, abgesehen von etwaigen Abstrahlungs- und Ableitungsverlusten, bevorzugt adiabat (d. h. ohne technische Maßnahmen zur Abfuhr der Reaktionswärme) umgesetzt. Dabei wird die adiabate Temperaturerhöhung der Mischvorrichtung und im Reaktionsraum allein über die Temperaturen, Zusammensetzungen und relativen Dosierungen der Eduktströme sowie der Verweilzeit in der Mischvorrichtung und dem Reaktionsraum eingestellt. Eine isotherme Umsetzung ist grundsätzlich auch denkbar, allerdings muss bei der Abfuhr der Reaktionswärme unbedingt darauf geachtet werden, dass es nicht zu einer vorzeitigen Kondensation kommt.

Die Verweilzeit im Reaktionsraum beträgt bevorzugt 0,05 s bis 15 s, besonders bevorzugt 0,5 s bis 5 s. Der Druck p4 (der Druck im Reaktionsraum) beträgt bevorzugt 0,08 bar bis 2,5 bar, besonders bevorzugt 0,12 bar bis 2,4 bar und ganz besonders bevorzugt 0,15 bar bis 2,2 bar. Die Umsetzung erfolgt bei einer Edukttemperatur von bevorzugt 200 °C bis 600 °C, besonders bevorzugt 201°C bis 500°C. Bevorzugt durchströmt das gasförmige Reaktionsgemisch den Reaktionsraum im Wesentlichen ohne Rückvermischung. Dies wird dadurch erreicht, dass die Reaktionsmischung während des Durchströmens des Reaktionsraums ein Druckgefälle durchläuft (siehe unten für weitere Details).

In einer bevorzugten Ausführungsform des erfindungsgemäßen Verfahrens beträgt die Durchsatzkapazität des eingesetzten Reaktors bei den erfindungsgemäß geforderten Reaktionsbedingungen > 1 t Diamin / h, bevorzugt 2 - 50 t Diamin / h, besonders bevorzugt 2 ― 18 t Diamin / h. Besonders bevorzugt gelten diese Werte für Toluylendiamin, 1,6-Diamino-hexan und für Isophorondiamin. Unter Durchsatzkapazität ist dabei zu verstehen, dass in dem Reaktor die genannte Durchsatzkapazität an Diamin pro Stunde umgesetzt werden kann.

Nach Durchlaufen des Reaktionsraums wird die Umsetzung abgebrochen, und zwar bevorzugt durch Abkühlung des Reaktionsgemisches auf eine Temperatur, bei der das gebildete Diisocyanat kondensiert, die jedoch noch oberhalb der Zersetzungstemperatur des entsprechenden Carbamylchlorids liegt.

Dies kann beispielsweise dadurch erfolgen, dass das den Reaktionsraum kontinuierlich verlassende Gemisch, umfassend wenigstens Diisocyanat, Phosgen und Chlorwasserstoff, nach Verlassen des Reaktionsraumes durch ein inertes Lösungsmittel geleitet wird, worin das Diisocyanat gelöst wird, wie es bereits für andere Gasphasenphosgenierungen empfohlen wurde **(**EP 0 749 958 A1).

Bevorzugt erfolgt der Reaktionsabbruch jedoch dadurch, dass der in dem erfindungsgemäßen Verfahren eingesetzte Reaktor zumindest eine Zone aufweist, in die zum Abbruch der Umsetzung der Amine und des Phosgens zu den entsprechenden Isocyanaten ein oder mehrere geeignete Flüssigkeitsströme ("Quench-Flüssigkeiten") eingesprüht werden. Dadurch kann, wie in EP 1 403 248 A1**,** Abschnitt [0009] bis [0019], beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Kühlzone in eine Quenchstufe, wie sie z. B. in den Abschnitten [0014] bis [0024], [0032] bis [0045] und [0048] von EP 403 248 A1 offenbart wurde, integriert. In einer besonders bevorzugten Form kommen mehrere Kühlzonen zum Einsatz, erfolgen Integration und Betrieb dieser zumindest zwei Kühlzonen mit einer Quenchstufe, wie hinsichtlich Aufbau und Betrieb in EP 1 935 875 A1 offenbart.

Statt des integrierten Verbundes von der zumindest einen Kühlzone eines Reaktors mit einer Quenchstufe, wie sie in der EP 1 935 875 A1 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

Unabhängig von der Art der gewählten Abkühlung wird die Temperatur der zumindest einen Abkühlzone bevorzugt so gewählt, dass sie einerseits oberhalb der Zersetzungstemperatur des dem Diisocyanat entsprechenden Carbamylchlorids liegt. Andererseits sollen Isocyanat und gegebenenfalls das in dem gasförmigen Aminstrom und / oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und ggf. als Verdünnungsmittel mit verwendetes Inertgas die Kondensations- bzw. Quenchstufe weitestgehend nicht kondensiert bzw. nicht gelöst durchlaufen. Zur selektiven Gewinnung des Diisocyanats aus dem gasförmigen Reaktionsgemisch besonders gut geeignet sind bei einer Temperatur von 80 °C bis 200 °C, vorzugsweise 80 °C bis 180 °C gehaltene Lösungsmittel wie beispielsweise Chlorbenzol und / oder Dichlorbenzol, oder in diesen Temperaturbereichen gehaltenes Diisocyanat oder Mischungen des Diisocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Diisocyanats in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quenchflüssigkeit löst.

Die Erzeugung der für das erfindungsgemäße Verfahren bevorzugten Strömung des gasförmigen Reaktionsgemischs als Strömung ohne wesentliche Rückvermischung durch den Reaktionsraum wird durch ein Druckgefälle über die Mischeinrichtung sichergestellt. Dabei ist das Druckgefälle über die Mischeinrichtung maßgeblich für die Aufrechterhaltung einer gerichteten Strömung und einer guten Vermischung der Edukte verantwortlich. Vom Austritt aus der Mischeinrichtung bis zur Kondensations- bzw. Quenchstufe liegt dagegen kein (signifikantes) Druckgefälle vor; hier herrscht überall der Druck p4. Die Drücke p2 (Aminstrom A vor Eintritt in die Mischvorrichtung) und p3 (Phosgenstrom P vor Eintritt in die Mischvorrichtung) sind also beide jeweils größer als p4, und zwar mindestens um den Betrag des Druckgefälles über die Mischeinrichtung.

Das die Kondensations- bzw. Quenchstufe verlassende Gasgemisch wird bevorzugt in einer nachgeschalteten Gaswäsche mit einer geeigneten Waschflüssigkeit von ggf. vorhandenem restlichem Diisocyanat befreit und bevorzugt anschließend in an sich bekannter Weise von überschüssigem Phosgen befreit. Dies kann mittels einer Kühlfalle, Absorption in einem inerten Lösungsmittel (z. B. Chlorbenzol oder Dichlorbenzol) oder durch Adsorption und Hydrolyse an Aktivkohle erfolgen. Bevorzugt wird das aus dem Gasgemisch abgetrennte überschüssige Phosgen wieder dem Reaktor zugeführt. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionsraumes eingesetzt.

Die Reindarstellung der Diisocyanate erfolgt bevorzugt anschließend durch destillative Aufarbeitung der Lösungen bzw. Gemische aus der Kondensations- bzw. Quenchstufe.

Durch das erfindungsgemäße Verfahren veringert sich die thermische Belastung des eingesetzten Diamins und damit auch seine partielle Zersetzung unter Ammoniakabspaltung. Da die Zersetzung des Diamins unter Ammoniakabspaltung zur Bildung von Anbackungen aus Ammoniumchlorid führt, wird durch das erfindungsgemäße Verfahren die Verschmutzung der Produktionsanlage deutlich reduziert. Dies wiederum führt zu einem stabileren Betrieb und längeren Laufzeiten. Im Gegensatz zu aus dem Stand der Technik bekannten Verfahren, bei denen unter Einsatz eines Diffusors Druck und Temperatur der Reaktionsmischung nach Vermischung von Amin und Phosgen erhöht werden, bietet das erfindungsgemäße Verfahren folgende Vorteile:
- Die Inertgasanteile der Eduktströme sind in jedem Bereich des dem Reaktionsraum zugeführten Diamin-Mengenstroms unabhängig voneinander einstellbar. Dadurch wird eine unerwünschte Gasbelastung der Anlage und eine aufwändige Ausschleusung bzw. Rückführung vermieden, und der Reaktionsraum kann bei verschiedenen Diamin-Mengenströmen im jeweils günstigen Bereich der Fluidströmung betrieben werden.
- Menge und Geschwindigkeit der Eduktströme sind voneinander unabhängig einstellbar. Dadurch wird der regeltechnische Aufwand verringert und die Anzahl möglicher Parametervariationen erhöht.
- Der Phosgendruck vor der Mischzone ist bei gegebenem Druck in der Reaktionszone geringer, da kein Treibstrahleffekt (wie bei einem Ejektor) erforderlich ist. Ein geringerer Vordruck ermöglicht, den Sicherheitsanforderungen im Umgang mit Phosgen besser gerecht zu werden.

### Beispiele:

### Beispiele 1 und 2:

20,5 kmol/h eines Gemisches aus 2,4- und 2,6-Toluylendiamin im Massenverhältnis von 4 : 1 werden in einen Verdampfer geleitet und in den gasförmigen Zustand überführt. Der Verdampfer ist als Fallfilmverdampfer mit Pumpvorlage und Umlaufpumpe ausgeführt und wird bei einer Temperatur T1 und einem Druck p1 betrieben. Das gasförmige Diamin gelangt in einen ersten Überhitzer mit Tropfenabscheider zur Rückführung nicht verdampfter Anteile in den Verdampfer. Das TDA wird dabei auf eine Temperatur T11 erhitzt. Das auf T11 erhitzte TDA wird in einem nächsten Schritt entweder in einen weiteren Überhitzer geleitet und dort auf T2 erhitzt (**Beispiel 1** - Vergleich) oder in einen Turboverdichter geleitet, wo der Druck des TDA auf p2 erhöht wird und die Temperatur des TDA auf T2 steigt (**Beispiel 2** - erfindungsgemäß). Danach wird der Amindampf durch eine auf der Reaktorachse angeordnete Glattstrahldüse in den rotationssymmetrischen Rohrreaktor geleitet. Gleichzeitig werden parallel dazu 123 kmol/h Phosgen mit 500 kg/h ortho-Dichlorbenzol verdampft und mit 320°C bei einem Druck von 1600 mbar (p3) über den die Glattstrahldüse umgebenden Ringraum dem Reaktor zugeführt. Im Reaktor herrscht der Druck p4. Alle Apparate und Rohrleitungen ab TDA- und Phosgen-Verdampfung bis einschließlich Reaktor sind entsprechend isoliert und wo erforderlich mit einer Begleitbeheizung versehen, um durch Wärmeverluste bedingte, unerwünschte Kondensation oder Ablagerungen zu vermeiden. Die Reaktion erreicht eine adiabatische Endtemperatur von 405°C und ist nach 5,5 Sekunden beendet. Das Reaktionsgasgemisch wird durch Eindüsen von ortho-Dichlorbenzol gekühlt (Quenche) und das gebildete Isocyanat kondensiert und ausgewaschen sowie anschließend nach bekannten Methoden destillativ aufgearbeitet.

Nach 165 Tagen Laufzeit wird die Anlage abgeschaltet und zwecks Reinigung und Wartung geöffnet.

Nachstehende Tabelle fasst die Versuchsparameter und Ergebnisse zusammen:

**Tabelle: Gegenüberstellung von Beispiel 1 (Vergleich) und Beispiel 2 (erfindungsgemäß)**

| **Beispie l** | **p1 / mbar** | **p2 / mbar** | **p3 / mbar** | **p4 / mbar** | **T1/ °C** | **T11 / °C** | **T2 / °C** | **Ausbeu te an TDI/ % der Th. ^{[1]}** | **Zustand der Anlage nach 165 d** |
|---|---|---|---|---|---|---|---|---|---|
| **1 (Vgl.)** | 1600 | - | 1600 | 1540 | 306 | 330 | 395 | 95,5 | [2] |
| **2** | 800 | 1600 | 1600 | 1555 | 278 | 360 | 395 | 95,7 | [3] |

| | | | | | | | | | |
|---|---|---|---|---|---|---|---|---|---|
| [1] Die Berechnung der Ausbeute bezieht sich auf die Umsetzung des eingesetzten TDA zu TDI in der Rohware (Sumpf nach Quenche). [2] An der Innenwandung des Reaktors im Bereich der Kondensationsstufe (Quenche) unterhalb der Quenchedüsen und im Sammelbehälter des Isocyanat-Rohproduktes nach der Quenche finden sich Anbackungen und Ablagerungen, die auf vermehrte Nebenproduktbildung zurückzuführen sind. Im Bereich der Aufarbeitung des die Kondensationsstufe verlassenden Gasgemisches werden in Apparaten und Rohrleitungen feste Ablagerungen, die überwiegend aus Ammoniumchlorid bestehen, gefunden. Betroffen sind größtenteils Oberflächen mit tieferen Temperaturen, die eine Kondensation von festem Ammoniumchlorid aus der Gasphase begünstigen. Ammoniumchlorid bildet sich aus Ammoniak und Chlorwasserstoff in der Gasphasenphosgenierung nach thermischer Zersetzung des Ausgangsamins. Diese Ammoniumchloridablagerungen erhöhen den Druckverlust in der Anlage, führen zur Verschlechterung von Wärmeübergängen sowie zur Beeinträchtigung von Stoffaustauschflächen entsprechender Einbauten. [3] Die Innenwandung und der Sammelbehälter des Isocyanat-Rohproduktes nach der Quenche sind im Wesentlichen frei von Ablagerungen. Auch im Bereich der Aufarbeitung Isocyanat-Rohgemisches und des die Kondensationsstufe verlassenden Gasgemisches werden keine nennenswerten Ablagerungen gefunden. | | | | | | | | | |

Wie man sieht, führt die erfindungsgemäße Vorgehensweise zu einer Vermeidung unerwünschter Ablagerungen und etwas erhöhter Ausbeute.

## Patentansprüche

1. Verfahren zur Herstellung eines Diisocyanats durch Umsetzung des korrespondierenden primären Diamins mit Phosgen in der Gasphase, worin
(i) das primäre Diamin getrennt vom Phosgen bei einem Druck p1 in die Gasphase überführt wird, sodass ein gasförmiger, das Diamin enthaltender Eduktstrom A erhalten wird,
(ii) der in (i) erhaltene Eduktstrom A, optional nach Überhitzung, durch ein Druckerhöhungselement geführt wird, in dem der Druck des Eduktstroms A auf einen Wert p2 > p1 erhöht wird,
(iii) ein Phosgen enthaltender gasförmiger Eduktstrom P in einer Mischvorrichtung mit dem Strom A aus Schritt (ii) vermischt wird, und
(iv) der in (iii) erhaltene Mischstrom aus den Eduktströmen A und P in einem Reaktionsraum zum Diisocyanat umgesetzt wird.

2. Verfahren nach Anspruch 1, bei dem das Druckerhöhungselement, das der Eduktstrom A durchläuft, ausgewählt ist aus der Gruppe bestehend aus:
Kolbenverdichter, Schraubenverdichter, Drehkolbenverdichter und Turboverdichter.

3. Verfahren nach einem der Ansprüche 1 oder 2, bei dem die Mischvorrichtung eine statische Mischvorrichtung ist.

4. Verfahren nach Anspruch 3, bei dem die statische Mischvorrichtung ausgewählt ist aus der Gruppe bestehend aus:
Glattstrahldüsen und Düsen mit turbulenzerzeugenden Elementen.

5. Verfahren nach einem der Ansprüche 1 bis 4, bei dem eine Druckdifferenz p2 - p1 von 50 mbar bis 1500 mbar eingestellt wird.

6. Verfahren nach einem der Ansprüche 1 bis 5, bei dem der Eduktstrom P bei Eintritt in die Mischvorrichtung unter einem Druck p3 steht, der größer als oder gleich p2 ist.

7. Verfahren nach Anspruch 6, bei dem eine Druckdifferenz p3 - p2 von bis zu 100 mbar eingestellt wird.

8. Verfahren nach einem der Ansprüche 1 bis 7, bei dem die Umsetzung in Schritt (iv) bei einem Druck p4 erfolgt, der kleiner als p2 und kleiner als p3 ist.

9. Verfahren nach Anspruch 8, bei dem die Druckdifferenzen p3 - p4 und p2 - p4 jeweils unabhängig voneinander 20 mbar bis 800 mbar, bevorzugt 20 mbar bis 500 mbar, besonders bevorzugt 30 mbar bis 400 mbar und ganz besonders bevorzugt 50 mbar bis 300 mbar betragen.

10. Verfahren nach einem der Ansprüche 1 bis 9, bei dem das in Schritt (iv) erhaltene Diisocyanat durch weitere Schritte umfassend eine Destillation zu gereinigtem Diisocyanat aufgearbeitet wird.

## Claims

1. Method for preparing a diisocyanate by reacting the corresponding primary diamine with phosgene in the gas phase wherein
(i) the primary diamine is transferred into the gas phase at a pressure p1 separately from the phosgene to obtain a gaseous starting material stream A comprising the diamine,
(ii) the starting material stream A obtained in (i) is led, optionally after superheating, through a pressure-raising element in which the pressure of starting material stream A is raised to a value p2 > p1,
(iii)a phosgene-containing gaseous starting material stream P is mixed with the stream A from step (ii) in a mixing device, and
(iv) the mixed stream obtained in (iii) out of said starting material streams A and P is converted in a reaction space into the diisocyanate.

2. Method according to Claim 1 wherein the pressure-raising element traversed by said starting material stream A is selected from the group consisting of: piston compressor, screw compressor, rotary piston compressor and turbocompressor.

3. Method according to either of Claims 1 and 2 wherein the mixing device is a static mixing device.

4. Method according to Claim 3 wherein the static mixing device is selected from the group consisting of:
smooth jet nozzles and nozzles incorporating turbulency-generating elements.

5. Method according to any one of Claims 1 to 4 wherein a pressure difference p2 ― p1 of 50 mbar to 1500 mbar is established.

6. Method according to any one of Claims 1 to 5 wherein said starting material stream P on entry into the mixing device is under a pressure p3 which is greater than or equal to p2.

7. Method according to Claim 6 wherein a pressure difference p3 - p2 of up to 100 mbar is established.

8. Method according to any one of Claims 1 to 7 wherein the reaction in step (iv) is effected at a pressure p4 which is less than p2 and less than p3.

9. Method according to Claim 8 wherein the pressure differences p3 - p4 and p2 - p4 each independently range from 20 mbar to 800 mbar, preferably from 20 mbar to 500 mbar, more preferably from 30 mbar to 400 mbar and most preferably from 50 mbar to 300 mbar.

10. Method according to any one of Claims 1 to 9 wherein the diisocyanate obtained in step (iv) is worked up to purified diisocyanate by further steps comprising a distillation.

## Revendications

1. Procédé de préparation d'un diisocyanate par réaction de la diamine primaire correspondante avec du phosgène en phase gazeuse, procédé dans lequel
(i) la diamine primaire est convertie en phase gazeuse séparément du phosgène à une pression p1 de façon à obtenir un flux d'éduit gazeux A contenant la diamine,
(ii) le flux d'éduit A obtenu en (i), éventuellement après surchauffe, est passé à travers un élément d'élévation de pression dans lequel la pression du flux d'éduit A est élevée à une valeur p2 > p1,
(iii) un flux d'éduit gazeux P contenant du phosgène est mélangé au flux A de l'étape (ii) dans un dispositif de mélange, et
(iv) le flux de mélange obtenu en (iii) à partir des flux d'éduit A et P est converti en diisocyanate dans une chambre de réaction.

2. Procédé selon la revendication 1, l'élément d'élévation de pression, qui est traversé par le flux d'éduit A, étant choisi dans le groupe comprenant :
les compresseurs alternatifs, les compresseurs à vis, les compresseurs à pistons rotatifs et les turbocompresseurs.

3. Procédé selon l'une des revendications 1 ou 2, dans lequel le dispositif de mélange est un dispositif de mélange statique.

4. Procédé selon la revendication 3, dans lequel le dispositif de mélange statique est choisi dans le groupe comprenant :
les buses à jet lisse et les buses à éléments générateurs de turbulences.

5. Procédé selon l'une des revendications 1 à 4, dans lequel une différence de pression p2 - p1 de 50 mbar à 1500 mbar est réglée.

6. Procédé selon l'une des revendications 1 à 5, dans lequel le flux d'éduit P est à une pression p3, qui est supérieure ou égale à p2, à l'entrée du dispositif de mélange.

7. Procédé selon la revendication 6, dans lequel une différence de pression p3 - p2 allant jusqu'à 100 mbar est réglée.

8. Procédé selon l'une des revendications 1 à 7, dans lequel la réaction de l'étape (iv) est effectuée à une pression p4 qui est inférieure à p2 et inférieure à p3.

9. Procédé selon la revendication 8, dans lequel les différences de pression p3 - p4 et p2 - p4 sont, indépendamment l'une de l'autre, de 20 mbar à 800 mbar, de préférence 20 mbar à 500 mbar, de manière particulièrement préférée 30 mbar à 400 mbar et tout particulièrement de 50 mbar à 300 mbar.

10. Procédé selon l'une des revendications 1 à 9, dans lequel le diisocyanate obtenu à l'étape (iv) est traité par des étapes supplémentaires comprenant une distillation pour donner du diisocyanate purifié.
